# EUROPEAN PATENT APPLICATION

(11) **EP 4 477 708 A2**
(43) Date of publication of application: **18.12.2024**
(21) Application number: 24202776.1
(22) Date of filing: 13.09.2021
(51) Int. Cl.: C08L 27/24

(54) **ALKALINE RESISTANT HALOGENATED PLASTIC FOR MEDICAL DEVICES**

(30) Priority: 14.09.2020 US 202017020479
(62) Divisional of application: 21787201.9
(71) Applicant: Carefusion 303 Inc., San Diego, California 92130 (US)
(72) Inventor: Meng, Fanqing, Buffalo Grove, 60089 (US); Weimer, Marc, South Jordan, 84095 (US); Cheng, Shan, Pine Brook, 07058 (US); Trainer, Lawrence, Murray, 84107 (US)
(74) Representative: Zenz Patentanwälte Partnerschaft mbB

(57) **Abstract**

Medical devices having an alkaline resistant halogenated polymer include a halogenated polymer incorporating one or more of a polymeric stabilizer, a polymeric plasticizer or a free radical inhibitor or combinations thereof. The medical devices can be used to administer high pH formulations to a patient in need of such administration by administering the formulation through contact the an alkaline resistant halogenated polymer.

## Description

### TECHNICAL FIELD

The present disclosure generally relates to halogenated plastics for medical devices and, in particular, to halogenated plastics for contact with high pH formulations such as tubing for administration of medical fluid by infusion.

### BACKGROUND

Plastic materials are extensively used in the medical field, particularly for patient diagnostic and treatment procedures. Polyvinyl chloride (PVC) is among the most widely used of such plastics and are commonly used with intravenous infusion devices to administer medicinal fluids. Many medicinal fluids have a pH that spans from acidic to approximately neutral, e.g., a pH of about 4 to about 7.4.

However, some drugs delivered by infusion medical devices can have extreme pH conditions. For instances, low pH formulations containing drugs such as Cefepime, Chlorpromazine, Metoclopramide and high pH formulations containing drugs of Flolan, Allopurinol, Ganciclovir and Methohexitol that range from pH of 2 to a pH of 12 from their excipient formulations. Such extreme pH fluids can adversely affect plastics in contact therewith.

Further, the plastics used for infusing medicinal fluids are typically sterilized by such routes as gamma, e-beam and ethylene oxide (EtO). Other sterilization procedures involve UV radiation, NO₂ gas, and wet heat/steam etc. Such sterilization methods can also adversely affect plastics used to administer medicinal fluids.

For example, PVC is known to degrade at high temperature with the evidence of discoloration and loss of physical and mechanical properties. Thermal degradation of PVC occurs by an autocatalytic dehydrochlorination reaction with the subsequent formation of conjugated double bonds during heat related processing of compounding and extrusion/injection molding. Stabilizers are thus used with PVC to improve thermal stability.

However, a continuing need exists to stabilize plastics used in medical devices particularly halogenated plastics subjected to high pH and/or harsh sterilization conditions.

### SUMMARY

Aspects of the subj ect technology relate to methods of administering a high alkaline pharmaceutical formulation having a pH greater than 7. Such alkaline pharmaceutical formulation can have a pH of no less than 8, e.g., no less than about 9 such as in a range of from about 7 to about 14 or about 8, or 9 to about 12 or 14. The methods include administering pharmaceutical formulation having a pH through contact with an alkaline resistant halogenated polymer.

Such formulations can be administered through direct contact with the alkaline resistant halogenated polymer and for prolonged periods of administration, e.g., the high alkaline formulation can contact the alkaline resistant halogenated polymer for more than one hour such as more than 4 hours and even over the period of days.

The alkaline resistant halogenated polymer advantageously can resist deleterious effects of the high alkaline formulation by incorporating one or more of a polymeric stabilizer, a polymeric plasticizer or a free radical inhibitor or a combination thereof with a halogenated polymer.

In some aspects, the alkaline resistant halogenated polymer can be in the form of tubing or a drip chamber. In other aspects, the alkaline resistant halogenated polymer can comprise a chlorinated polymers, e.g., PVC. In further aspects, the alkaline resistant halogenated polymer is subjected to sterilization by radiation prior to contact with the high alkaline pharmaceutical formulation.

Embodiments include one or more of the following features individually or combined. For example, the polymeric stabilizer can comprise one or more of an ion exchange resin, or an ionomer. The polymeric plasticizer can comprise one or more of an aliphatic polyester or one or more of an aliphatic polyester. The free radical inhibitor can comprise one or more of hydroquinone or derivative thereof. Further, the alkaline resistant halogenated polymer can comprise a non-dialkylester based plasticizer.

Additional advantages of the subject technology will become readily apparent to those skilled in this art from the following detailed description, wherein only certain aspects of the subject technology are shown and described, simply by way of illustration. As will be realized, the subject technology is capable of other and different configurations, and its several details are capable of modifications in various other respects, all without departing from the subject technology. Accordingly, the drawings and description are to be regarded as illustrative in nature, and not as restrictive.

### DETAILED DESCRIPTION

The detailed description set forth below describes various configurations of the subject technology and is not intended to represent the only configurations in which the subject technology may be practiced. The detailed description includes specific details for the purpose of providing a thorough understanding of the subject technology. Accordingly, dimensions are provided in regard to certain aspects as non-limiting examples. However, it will be apparent to those skilled in the art that the subject technology may be practiced without these specific details. In some instances, well-known structures and components are shown in block diagram form in order to avoid obscuring the concepts of the subject technology.

It is to be understood that the present disclosure includes examples of the subject technology and does not limit the scope of the appended claims. Various aspects of the subject technology will now be disclosed according to particular but non-limiting examples. Various embodiments described in the present disclosure may be carried out in different ways and variations, and in accordance with a desired application or implementation.

Aspects of the subj ect technology relate to medical devices that can come in contact with high pH pharmaceutical formulations. Such devices include, for example, medical tubing, drip chambers, etc. Medical treatments often include the infusion of a medical fluid (e.g., a saline solution or a liquid medication) to patients using an intravenous (IV) catheter that is connected though an arrangement of flexible tubing and fittings, commonly referred to as an "IV set," to a source of fluid, for example, an IV bag. Such medical tubing and other devices of the infusion assembly can be made from a halogenated polymer, such as polyvinyl chloride.

Such medical devices are typically sterilized by such routes as gamma, e-beam and ethylene oxide (EtO). Other sterilization procedures involve UV radiation, NO₂ gas, and wet heat/steam etc. Such sterilization methods can adversely affect plastics use to administer medicinal fluids. It was found that administering a high alkaline pharmaceutical formulation can cause precipitation of substances from plasticized PVC tubing.

Formulation development studies showed that there are multiple factors that impact the alkaline resistance ability of PVC tubing. Among those factors, the stabilizers ratio, their loading concentration and plasticizer type and loading level are sensitive factors to influence PVC high pH resistance. However, we were not able to completely eliminate the particulates by optimizing PVC formulations with conventional stabilizers and conventional plasticizers. Further, a larger quantity of particulates were observed upon radiation sterilization of gamma or e-beam compared to non-sterile PVC.

To improve the alkaline resistance of halogenated polymers used with medical devices, one or more of a polymeric components or free radical stabilizers can be incorporated with the halogenated polymer. Hence, an aspect of the subject technology relate to an alkaline resistant halogenated polymer formulated with one or more of a polymeric stabilizer, a polymeric plasticizer or a free radical inhibitor. Such alkaline resistant halogenated polymer can be used to administer a high alkaline pharmaceutical formulation having a pH greater than 7 such as no less than 8, e.g., no less than about 9 such as in a range of from about 7 to about 14 or about 9 to about 12, to a patient in need of such a formulation. Such formulations can be administered through direct contact with the alkaline resistant halogenated polymer and for prolonged periods of administration, e.g., the high alkaline formulation can contact the alkaline resistant halogenated polymer for more than one hour such as more than 4 hours and even over the period of days.

Halogenated polymers that can be formulated with one or more of a polymeric stabilizer, a polymeric plasticizer or a free radical inhibitor to form an alkaline resistant halogenated polymer include, for example, chlorinated polymers such as polyvinyl chloride (PVC), poly(vinylidene chloride), fluorinated polymers such as poly(vinylidene fluoride), poly(tetrafluoroethylene), mixed halogenated polymers such as poly(chlorotrifluroethylene), etc.

An aspect of the present disclosure includes medical tubing and drip chambers comprising polyvinyl chloride as a halogenated polymer incorporating one or more of a polymeric stabilizer, a polymeric plasticizer or a free radical inhibitor to improve high pH alkaline extraction resistance for post radiation sterilization applications

Useful polymeric stabilizers include one or more of an ion-exchange resin such as a cationic exchange resin, and/or one or more of an ionomer. Cationic exchange resin can have a structure with sub-micro to nano-scaled size of particles as HCl scavenger. Ion-exchange resins are generally made from methacrylic acid, sulfonated styrene, and divinylbenzene (DVB) and the generic structures are shown in scheme 1 and 2 below, respectively.

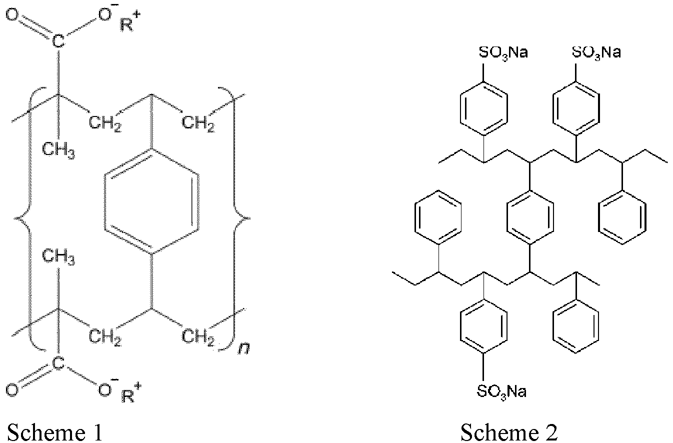

Such ion exchange resins typically include metal cations such as sodium cations. The ion-exchange resin can be immobilized in a crosslinked 3D network and its diffusion is significantly limited in a localized halogenated polymer matrix thus minimizing diffusion of the resin into an aqueous formulation. The ability of an ion-exchange resin such as a cationic exchange resin to stabilize a halogenated polymer such as PVC will depend, in part, on the acid absorption capacity, the particle size/surface area and loading level of the resin in the halogenated polymer. In an aspect of the present disclosure, the metal cation of the ion exchange resin can include Li, Na, K and other elements in group I, or Mg, Ca and elements in group II of Period table as well as other transition metal such of Zinc, Cd, Ni etc. An example is Dow's Dowex brand.

Alternatively, a polymeric thermal stabilizer that can be incorporated in a halogenated polymer to form an alkaline resistant halogenated polymer can be a metal salt of an organic polymer, e.g., an ionomer. In one embodiment, the ionomer is a metal salt from an acrylic acid containing polymer or a metal salt from a sulfonated polystyrene. The metal cation of the ionomer can be one or more of lithium cations, sodium cations, potassium cations, calcium cations, barium cations, and zinc cations, for example. Additional ionomers useful to include in the alkaline resistant halogenated polymer of the present disclosure can be found in U.S. Patent No. 5328948, which is incorporated herein by reference.

In addition to, or as a substitute to low molecular weight plasticizers, alkaline resistant halogenated polymers of the present disclosure can include one or more of a polymeric plasticizer. Useful polymeric plasticizers that can be used in the alkaline resistant halogenated polymers of the present disclosure include one or more of an aliphatic polyester, such as a polyadipate, a bio-based polymeric plasticizers such as a plasticizer based on a plant oil such as castor oil. Table 1 below lists a few polymeric plasticizers that can be incorporated in a halogenated polymer to form an alkaline resistant halogenated polymer of the present disclosure.

As described in the table below, polyadipates are aliphatic, medium to ultra-high molecular weight polyesters formed from polyalcohols and adipic acid by polycondensation. These linear or lightly branched polyester polyols have a low melting and glass transition temperature, high resistance to solvents and flames, but relatively poor hydrolytic stability due to the intrinsic ester functionality. However, it is expected that even a partially degraded fragment will have slow migration due to its high molecular weight. For example, high molecular weight polyesters have a very low potential for migration into a lipophilic substance. A recent study that measured extraction of di(2-ethylhexyl) phthalate (DEHP) and polyadipate from PVC nasogastric tubes through juice and feeding solution, found that polyadipate leaching was 10 times lower than that of DEHP in the feeding solution group and 100 times lower in the gastric juice group. See Van Vliet ED, et al. "A review of alternatives to di (2-ethylhexyl) phthalate-containing medical devices in the neonatal intensive care unit", J. Perinatol, 2011 August, 31(8): 551-560.

**Table 1 polymeric plasticizer and bio- based plasticizer**

| Category | Name | CAS |
|---|---|---|
| Polyadipates are polyesters of aliphatic dicarboxylic acids with diol | Hexanedioic acid, polymer with 2,2-dimethyl-1,3-propanediol and 1,2-propanediol, isononyl ester | 208945-13-5 |
| | Hexanedioic acid, polymer with 1,2-propanediol, octyl ester | 82904-80-1 |
| | Hexanedioic acid, polymer with 1,2-propanediol, acetate | 55799-38-7 |
| Bio-based | Soft-n-Safe is a castor oil based product | 736150-63-3 |

Three common polymeric adipates are polyethylene adipate, polypropylene adipate, and polybutylene adipate and can be illustrated by Scheme 3 below. A commercial available polyadipate for example is Palamoll^{®} Polymeric Plasticizers from BASF.

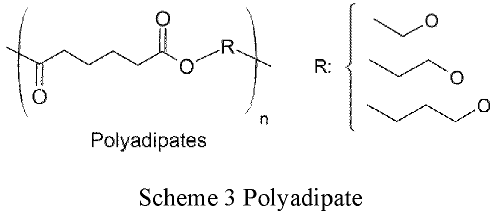

Another useful plasticizer that can be incorporated in a halogenated polymer to form an alkaline resistant halogenated polymer of the present disclosure includes non-dialkylester based plasticizer. Such non-dialkylester based plasticizers can increase the hydrolytic stability of a halogenated polymer in contact with a formulation with a high pH. Hence, in addition to or as a substitute to low molecular weight plasticizers, alkaline resistant halogenated polymers of the present disclosure can include one or more non-dialkylester based plasticizer. Such non-dialkylester based plasticizers include epoxidized cardanol diethyl phosphate which can be prepared from cardanol as illustrated in Scheme 4 below. See Puyou Jia, Haoyu xia etc. Plasticizers derived from biomass resources: a short review. Poymers 2018, 10, 1303. Such non-dialkylester based plasticizers can enhance the thermal stability of PVC, for example.

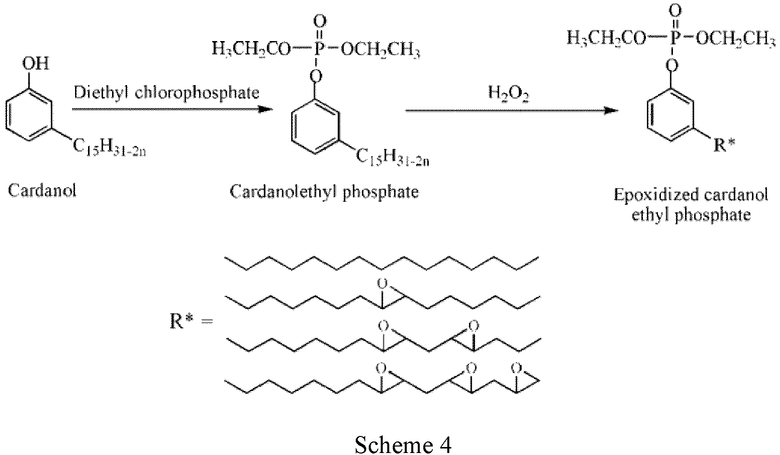

Other non-dialkylester based plasticizers that can be incorporated in a halogenated polymer to form an alkaline resistant halogenated polymer of the present disclosure include Alkylsulfonic acid phenylesters (ASEs), Sulfonamides, such as N-ethyl toluene sulfonamide, N-(2-hydroxypropyl) benzene sulfonamide, N-(n-butyl) benzene sulfonamide.

Alkaline resistant halogenated polymer of the present disclosure can include one or more of a free radical inhibitor. Free radical inhibiters are not commonly incorporated in halogenated polymers such as PVC. However, it was discovered that use of a free radical inhibitor can improve the alkaline resistance of halogenated polymers subjected to sterilization such as radiation sterilization.

During radiation sterilization processing, the molecular backbone or any liable atom might be dissociated to generate radicals which can continue to propagate and react until they are quenched by recombination or termination. To minimize this process, a radical inhibitor can be added to a halogenated polymer to terminate free radicals. Common free-radical inhibitors are phenolic compounds, such as hydroquinone (HQ), 4-Methoxyphenol (MEHQ) and their derivatives. Scheme 5 below shows the structure for MEHQ.

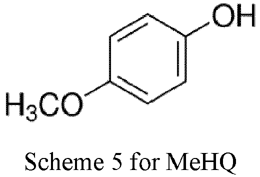

Such free radical inhibitors can be incorporated into a halogenated polymer, e.g., PVC, at concentrations approximately between 1 parts per hundred resin (phr) to 0.01 phr such as than less than 0.1 phr to prevent the radiation damage at the molecular level by mitigating free radical propagation.

### EXAMPLE

A commercially available PVC tubing that can be used with IV infusion sets was tested by extracting the tubing with a sodium hydroxide with phosphate buffer solution having a pH 11 for up to 7 days. The PVC tubing was plasticized with di(2-ethylhexyl) adipate (DEHA), di-2-ethylhexyl phthalate (DEHP) and Tris(2-ethylhexyl) trimellitate (TOTM) with the hardness from 70 shore A to 90 Shore A range, and contained zinc and calcium carboxylate stabilizers. It was observed that white particulates formed during the extraction process. Chemical analysis studies in DEHA plasticized PVC demonstrated that the particulates comprise of the plasticizer DEHA and its fragment 2-ethyl-hexanol, stearic and palmitic acid. A summary of the major ingredients of the particulates are provided in Table 2 below.

**Table 2**

| Component | Comment |
|---|---|
| DEHA | Low molecular weight plasticizer in PVC tube |
| 2-ethyl-1-hexanol | DEHA fragment |
| Palmitic Acid | Stabilizer fragment |
| Stearic acid | Stabilizer fragment |

As shown by the table above, exposing a commercial PVC tubing to a high alkaline solution results in degradation of the low molecular weight stabilizer and fragments of which are observed. Also observed are fragments from the stabilizer by way of palimitic acid and stearic acid. It is believed that the zinc and calcium carboxylate stabilizers in the PVC tubing have some level mobility and diffuse to the surface of the tubing when the tubing contacts a metal die during extrusion formation of tubing and when tubing is exposed to radiation sterilization. The carboxylate counterpart of the zinc and calcium carboxylate stabilizers (having 8 to 18 carbons chain) can dissociate from tubing surfaces to form an immiscible aggregation of solid precipitate. The ester plasticizer with small molecular weight in a harsh processing conditions are liable to hydrolysis at high pH conditions and form a polar head and non-polar tail surfactant structure. As summarized in the table above, chemical analytical study has shown that the white precipitate is composed of stearic acid and palmitic acid from the stabilizer and 2-ethyl-1-hexanol from DEHA plasticizer.

It is uncommon to add free radical inhibiter in PVC compounded resin. However, it is believed that including one or more free radical inhibiters in a halogenated polymer such as PVC would reduce degradation thereof when the halogenated polymer is subjected to heat when forming a medical device with the material and/or during sterilization. Subjecting such heated/sterilized halogenated polymers make them more susceptible to degradation when in contact with high pH fluids.

In summary, an alkaline resistant halogenated polymer comprising one or more halogenated polymers incorporating one or more of a polymeric stabilizer, a polymeric plasticizer or a free radical inhibitor can minimize degradation and diffusion during heating and radiation sterilization and permit the material to resist degradation such as forming precipitates when in prolonged contact with high alkaline pharmaceutical formulations.

It is understood that any specific order or hierarchy of blocks in the methods of processes disclosed is an illustration of example approaches. Based upon design or implementation preferences, it is understood that the specific order or hierarchy of blocks in the processes may be rearranged, or that all illustrated blocks be performed. In some implementations, any of the blocks may be performed simultaneously.

The present disclosure is provided to enable any person skilled in the art to practice the various aspects described herein. The disclosure provides various examples of the subject technology, and the subject technology is not limited to these examples. Various modifications to these aspects will be readily apparent to those skilled in the art, and the generic principles defined herein may be applied to other aspects.

A reference to an element in the singular is not intended to mean "one and only one" unless specifically so stated, but rather "one or more." Unless specifically stated otherwise, the term "some" refers to one or more. Pronouns in the masculine (e.g., his) include the feminine and neuter gender (e.g., her and its) and vice versa. Headings and subheadings, if any, are used for convenience only and do not limit the invention.

The word "exemplary" is used herein to mean "serving as an example or illustration." Any aspect or design described herein as "exemplary" is not necessarily to be construed as preferred or advantageous over other aspects or designs. In one aspect, various alternative configurations and operations described herein may be considered to be at least equivalent.

As used herein, the phrase "at least one of" preceding a series of items, with the term "or" to separate any of the items, modifies the list as a whole, rather than each item of the list. The phrase "at least one of" does not require selection of at least one item; rather, the phrase allows a meaning that includes at least one of any one of the items, and/or at least one of any combination of the items, and/or at least one of each of the items. By way of example, the phrase "at least one of A, B, or C" may refer to: only A, only B, or only C; or any combination of A, B, and C.

A phrase such as an "aspect" does not imply that such aspect is essential to the subject technology or that such aspect applies to all configurations of the subject technology. A disclosure relating to an aspect may apply to all configurations, or one or more configurations. An aspect may provide one or more examples. A phrase such as an aspect may refer to one or more aspects and vice versa. A phrase such as an "embodiment" does not imply that such embodiment is essential to the subject technology or that such embodiment applies to all configurations of the subject technology. A disclosure relating to an embodiment may apply to all embodiments, or one or more embodiments. An embodiment may provide one or more examples. A phrase such an embodiment may refer to one or more embodiments and vice versa. A phrase such as a "configuration" does not imply that such configuration is essential to the subject technology or that such configuration applies to all configurations of the subject technology. A disclosure relating to a configuration may apply to all configurations, or one or more configurations. A configuration may provide one or more examples. A phrase such a configuration may refer to one or more configurations and vice versa.

In one aspect, unless otherwise stated, all measurements, values, ratings, positions, magnitudes, sizes, and other specifications that are set forth in this specification, including in the claims that follow, are approximate, not exact. In one aspect, they are intended to have a reasonable range that is consistent with the functions to which they relate and with what is customary in the art to which they pertain.

It is understood that the specific order or hierarchy of steps, operations or processes disclosed is an illustration of exemplary approaches. Based upon design preferences, it is understood that the specific order or hierarchy of steps, operations or processes may be rearranged. Some of the steps, operations or processes may be performed simultaneously. Some or all of the steps, operations, or processes may be performed automatically, without the intervention of a user. The accompanying method claims, if any, present elements of the various steps, operations or processes in a sample order, and are not meant to be limited to the specific order or hierarchy presented.

All structural and functional equivalents to the elements of the various aspects described throughout this disclosure that are known or later come to be known to those of ordinary skill in the art are expressly incorporated herein by reference and are intended to be encompassed by the claims. Moreover, nothing disclosed herein is intended to be dedicated to the public regardless of whether such disclosure is explicitly recited in the claims. No claim element is to be construed under the provisions of 35 U.S.C. §112 (f) unless the element is expressly recited using the phrase "means for" or, in the case of a method claim, the element is recited using the phrase "step for." Furthermore, to the extent that the term "include," "have," or the like is used, such term is intended to be inclusive in a manner similar to the term "comprise" as "comprise" is interpreted when employed as a transitional word in a claim.

The Title, Background, Summary, Brief Description of the Drawings and Abstract of the disclosure are hereby incorporated into the disclosure and are provided as illustrative examples of the disclosure, not as restrictive descriptions. It is submitted with the understanding that they will not be used to limit the scope or meaning of the claims. In addition, in the Detailed Description, it can be seen that the description provides illustrative examples and the various features are grouped together in various embodiments for the purpose of streamlining the disclosure. This method of disclosure is not to be interpreted as reflecting an intention that the claimed subject matter requires more features than are expressly recited in each claim. Rather, as the following claims reflect, inventive subject matter lies in less than all features of a single disclosed configuration or operation. The following claims are hereby incorporated into the Detailed Description, with each claim standing on its own as a separately claimed subject matter.

The claims are not intended to be limited to the aspects described herein, but are to be accorded the full scope consistent with the language claims and to encompass all legal equivalents. Notwithstanding, none of the claims are intended to embrace subject matter that fails to satisfy the requirement of 35 U.S.C. § 101, 102, or 103, nor should they be interpreted in such a way.

In this writing the following concepts are disclosed:
1. A method of administering a high alkaline pharmaceutical formulation having a pH greater than 7, the method comprising administering the formulation through contact with an alkaline resistant halogenated polymer,
   wherein the alkaline resistant halogenated polymer comprises a halogenated polymer incorporating one or more of a polymeric stabilizer, a polymeric plasticizer or a free radical inhibitor.
2. The method of concept 1, wherein the high alkaline pharmaceutical formulation has a pH in the range of greater than 7 to 14 and the high alkaline formulation contacts the alkaline resistant halogenated polymer for more than one hour.
3. The method of concept 1, wherein the alkaline resistant halogenated polymer was sterilized by radiation prior to contact with the high alkaline pharmaceutical formulation.
4. The method of concept 3, wherein the alkaline resistant halogenated polymer comprises polyvinyl chloride as the halogenated polymer.
5. The method of concept 1, comprising administering the formulation through contact with tubing comprising the alkaline resistant halogenated polymer.
6. The method of concept 5, wherein the alkaline resistant halogenated polymer comprises polyvinyl chloride as the halogenated polymer.
7. The method of concept 6, wherein the alkaline resistant halogenated polymer was sterilized by radiation prior to contact with the high alkaline pharmaceutical formulation.
8. The method of concept 1, wherein the alkaline resistant halogenated polymer comprises polyvinyl chloride as the halogenated polymer.
9. The method of concept 1, wherein the halogenated polymer is a chlorinated polymers.
10. The method of concept 1, wherein the polymeric stabilizer comprises one or more of an ion exchange resin or an ionomer.
11. The method of concept 1, wherein the polymeric plasticizer comprises one or more of an aliphatic polyester.
12. The method of concept 1, wherein the free radical inhibitor comprises one or more of hydroquinone or derivative thereof.
13. The method of concept 1, wherein the alkaline resistant halogenated polymer comprises a non-dialkylester based plasticizer.
14. The method of concept 1, wherein the formulation contacts the alkaline resistant halogenated polymer for more than one hour.
15. A medical device comprising an alkaline resistant halogenated polymer comprising a halogenated polymer incorporating one or more of a polymeric stabilizer, a polymeric plasticizer or a free radical inhibitor or combinations thereof.
16. The medical device according to concept 15, wherein the halogenated polymer comprises polyvinyl chloride.
17. The medical device according to concept 16, wherein the halogenated polymer comprises polyvinyl chloride and in the form of a tube or a drip chamber.
18. The medical device according to concept 17, wherein the alkaline resistant halogenated polymer was sterilized by radiation.
19. The medical device according to concept 15, wherein the alkaline resistant halogenated polymer is in contact with a high alkaline pharmaceutical formulation having a pH greater than 7.
20. The medical device according to concept 15, wherein the alkaline resistant halogenated polymer is in contact with a high alkaline pharmaceutical formulation having a pH in the range of 8 to 14.

## Claims

1. An alkaline resistant halogenated polymer comprising a halogenated polymer incorporating a free radical inhibitor, a polymeric stabilizer, and a polymeric plasticizer, wherein the alkaline resistant halogenated polymer is sterilized by radiation prior to contact with a high alkaline pharmaceutical formulation.

2. The alkaline resistant halogenated polymer of claim 1, wherein the alkaline resistant halogenated polymer comprises polyvinyl chloride as the halogenated polymer.

3. The alkaline resistant halogenated polymer of claim 1, wherein the halogenated polymer is a chlorinated polymer.

4. The alkaline resistant halogenated polymer of any of the preceding claims, wherein the polymeric stabilizer comprises one or more of an ion exchange resin or an ionomer.

5. The alkaline resistant halogenated polymer of any of the preceding claims, wherein the polymeric plasticizer comprises one or more of an aliphatic polyester.

6. The alkaline resistant halogenated polymer of any of the preceding claims, wherein the free radical inhibitor comprises one or more of hydroquinone or derivative thereof.

7. The alkaline resistant halogenated polymer of any of the preceding claims, wherein the alkaline resistant halogenated polymer further comprises a non-dialkylester based plasticizer.

8. The alkaline resistant halogenated polymer of any of the preceding claims, wherein the free radical inhibitor comprises one or more of hydroquinone or derivative thereof.

9. The alkaline resistant halogenated polymer of any of the preceding claims, wherein the sterilizing radiation comprises exposure to ultraviolet radiation.

10. The alkaline resistant halogenated polymer of claim 1, wherein the halogenated polymer comprises one of a chlorinated polymer, a fluorinated polymer and a mixed halogenated polymer.

11. The alkaline resistant halogenated polymer of claim 3, wherein the chlorinated polymer comprises polyvinyl chloride (PVC) or poly(vinylidene chloride).

12. The alkaline resistant halogenated polymer of claim 10, wherein the fluorinated polymer comprises poly(vinylidene fluoride) or poly(tetrafluoroethylene).

13. The alkaline resistant halogenated polymer of claim 10, wherein the mixed halogenated polymer comprises poly(chlorotrifluroethylene).
